# EUROPEAN PATENT SPECIFICATION

(11) **EP 2 099 461 B1**
(45) Date of publication and mention of the grant of the patent: **28.03.2012**
(21) Application number: 06805565.6
(22) Date of filing: 13.11.2006
(51) Int. Cl.: A61K 31/7064, C07H 19/10, C07H 19/20, A61P 35/00, A61K 31/7076

(54) **LNA NUCLEOSIDE PHOSPHORAMIDATES**
LNA-NUKLEOSID-PHOSPHORAMIDATE
PHOSPHORAMIDATES DU NUCLÉOSIDE LNA

(43) Date of publication of application: 16.09.2009
(73) Proprietor: Santaris Pharma A/S, 2970 Hørsholm (DK)
(72) Inventor: ROSENBOHM, Christoph, 3460 Birkerød (DK); JENSEN, Jacob, DK-2300 Copenhagen S (DK); KOCH, Troels, DK-2300 Copenhagen S (DK)
(74) Representative: Turner, Mark Frederic Paris
(86) International application number: PCT/DK2006/000627
(87) International publication number: WO 2007/054100

(56) References cited:
- WO-A-03/039523
- WO-A-2005/012327
- BRYLD T ET AL: "Synthesis and antiviral evaluation of novel conformationally locked mucleosides and masked 5'-phosphate derivatives thereof" JOURNAL OF THE CHEMICAL SOCIETY, PERKIN TRANSACTIONS 1, CHEMICAL SOCIETY. LETCHWORTH, GB, vol. 1, 2002, pages 1655-1662, XP002252484 ISSN: 0300-922X cited in the application
- JACOB JENSEN, ET AL.: "Synthesis and biological evaluation of LNA Phophoramidates", NUCLEOSIDES, NUCLEOTIDES AND NUCLEIC ACIDS, vol. 27, 2007, pages 37-42, XP009128685,

## Description

### FIELD OF THE INVENTION

The present invention discloses compounds that are LNA nucleoside phosphoramidates which act as inhibitors of cell proliferation and provides the use of LNA nucleoside phosphoramidates in medicine.

### BACKGROUND OF THE INVENTION

Nucleoside analogues such as fluorodeoxyuridine, cytarabine and gemcitabine are well established as anticancer agents. They function as inhibitors of DNA synthesis after activation to their 5'-phosphate form.

The chemotherapeutic effect of NA's depends on their ability to penetrate into the cells and for some NA also to be phosphorylated (to their triphosphates) intracellularly. The free bioactive phosphate forms do not in general represent useful drugs due to their poor membrane permeation. This poses two problems; first the transformation from nucleoside to monophosphates is often a highly controlled process and therefore the key step in the intracellular conversion of the NA.[McGuigan, C.; Kinchington, D.; Nicholls, S. R.; Nickson, C.; O'Connor, T. J. Bloorg.Med.Chem.Lett. 1993, 3, 1207-1210.]. Second NA's phosphates are relatively polar compounds, which often limit their ability to penetrate the cell membrane

The use of lipophilic monophosphate prodrugs of NA's such as CycloSal, SATE, and phosphoramidates [Cahard, D.; McGuigan, C.; Balzarini, J. Mini-Reviews in Med.Chem. 2004, 4, 371-381] have in many cases proven be the solution to both problems, and the research within this field is progressing.

The last few years has seen the development of quite a few LNA (Locked Nucleic Acid) nucleosides but with a few exceptions [Bryld, T.; Sørensen, M. H.; Nielsen, P.; Koch, T.; Wengel, J. J.Chem.Soc., Perkin Trans.1 2002, 1655-1662.] they have only been used as building blocks for oligonucleotides in antisense treatment.

LNA nucleotides, as therapeutic agent In their own right, remains a rather unexplored area. We have therefore synthesised a series of lipophilic prodrugs of different LNA analogues in combination with the phosphoramidite approach.

Detailed descriptions on methods of preparation of LNA monomers and manipulations of suitable protecting groups In such compounds can be found In WO/2003095476.

Detailed descriptions on methods of preparation of phosphoramidates. In general and various variations In the ester can be found in [McGuigan et al, AVCC, 1998,9, 473-9], variations in amino acid In [McGuigan et al, Antiviral Res. , 1997,35, 195-204], and variations in aryl in [Siddiqul et al, J. Med. Chem., 1999, 42, 393-9] regions of the phosphoramidate, and nucleoside (McGuigan et al, BioOrg. Med, Chem. Lett. , 1996,6, 2369-72; McGuigan et al, Bioorg. Med. Chem. Lett., 2000,10, 645-7]. This work has lead to the optimal description of phenyl methoxyalaninyl phosphoramidate as the prototype pro-moiety for the intracellular delivery of bioactive nucleotides (Balzarini et al, PNAS, 1996,93, 7295-9; McGuigan et al, J. Med. Chem. 1996,39, 1748-53].

Certain phosphoramiditates conjugated to LNA nucleosides have been prepared and tested against against HIV *in vitro.* But none of the analogues prepared had activity against HIV (Bryld et al., J. Chem. Soc., Perkin Trans. 1, 2002, 1655-1662).

The present invention is based upon the surprising discovery that despite the absence of any activity against HIV, LNA nucleoside/phoshoramidate conjugates have a marked activity against cancer cells, and are therefore suited for use In medicine, particularly in the treatment of cancer.

### SUMMARY OF THE INVENTION

The present invention provides compounds according to claim 1 for use as a medicament

The present invention further discloses various methods of treatment/therapy comprising administering a compound of formula I to a patient, as well as the use of a compound of Formula **I** in the manufacture of a medicament for the treatment or prophylaxis of cancer.

The present invention further provides a compound of formula **I**, as herein defined, wherein R¹ is benzyl.

The present invention further provides a compound according to claim 14,

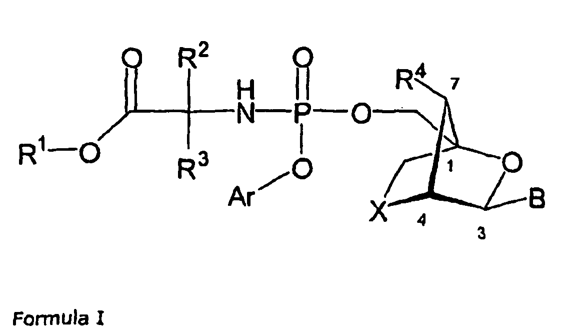

In one embodiment said compound is not selected from one of the following:

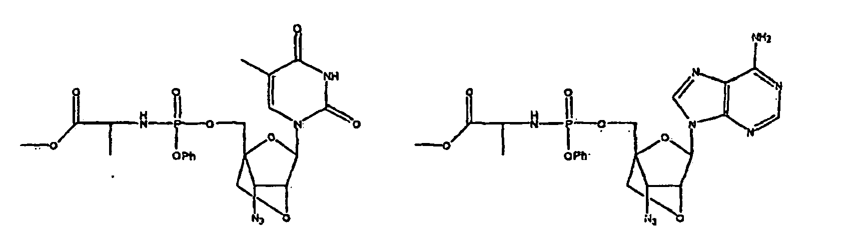

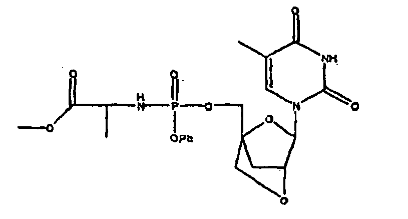

### BRIEF DESCRIPTION OF THE FIGURES

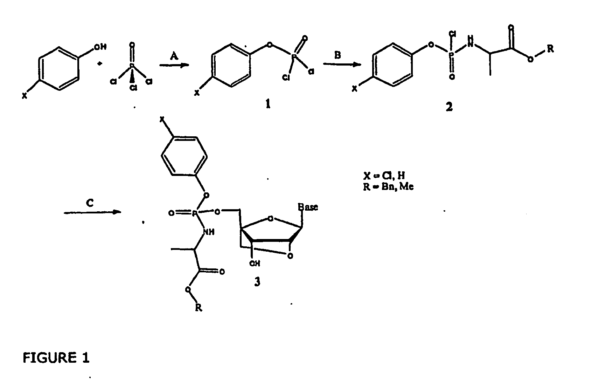

Figure 1 illustrates the general synthesis of the compounds of this invention: A) Et₃N, Et₂O, - 78°C, 5-12 h; B) Et₃N, L-alanine methyl/benzyl ester hydrochloride, DCM, -78°C to RT, 2-5 h; C) LNA nucleoside 1-4, ^{t}BuMgCl, Pyr/MeCN 2:1, 2 days.
Figure 2 illustrates the phosphoramidates synthesized.

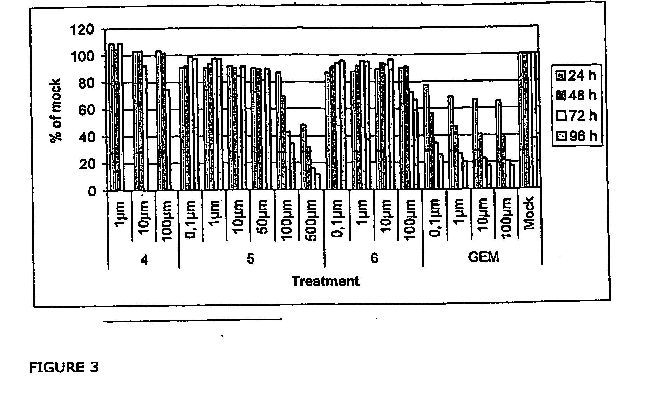

Figure 3 illustrates MTS assay data of selected LNA phosphoramidates and Gemcitabine (GEM) (positive control) normalised to Mock (blank).

### DESCRIPTION OF THE INVENTION

### Definitions

In the present context, the term "C₁-C₁₀ alkyl" is intended to mean a linear or branched hydrocarbon group having 1 to 10 carbon atoms, such as methyl, ethyl, propyl, *iso*propyl, cyclopropyl, butyl, *iso*-butyl, *tert*-butyl, cyclobutyl, pentyl, cyclopentyl, hexyl, cyclohexyl, etc. The cyclic variants hereof are often referred to as "cycloalkyl", more particular "C₃-C₁₀ cycloalkyl". "C₁-C₆ alkyl" (which is often preferred) - of course - refers to shorter variants having 1 to 6 carbon atoms.

Similarly, the term "C₂-C₆ alkenyl" is intended to cover linear, cyclic or branched hydrocarbon groups having 2 to 6 carbon atoms and comprising one unsaturated double bond. Examples of alkenyl groups are vinyl, allyl, butenyl, pentenyl, hexenyl, heptenyl, octenyl, hepta-decaenyl. Preferred examples of alkenyl are vinyl, allyl, butenyl, especially allyl. The term "C₁-C₆ alkynyl" is intended to cover linear; cyclic or branched hydrocarbon groups having 2 to 6 carbon atoms and comprising one unsaturated triple bond.

Moreover, the term "C₁-C₆ alkylene" is intended to mean a linear, cyclic or branched hydrocarbon biradical having 1 to 6 carbon atoms, such as methylene, ethylene, propylene, *iso*-propylene, cyclopropylene, butylene, *iso*-butylene, *tert*-butylene, cyclobutylene, pentylene, cyclopentylene, hexylene, cyclohexylene, etc. The cyclic variants hereof are often referred to as "cycloalkylene", more particular "C₃-C₆ cycloalkylene".

The terms "haloalkyl" and "haloalkylene" are intended to mean alkyl and alkylene, respectively, being substituted with one or more halogen atoms, e.g. one, two, three or four halogen atoms, or even halogen atoms corresponding to all hydrogen atoms of the alkylene (perhalogenation).

The term "acyl" means alkylcarbonyl, e.g. "C₁-C₆ acyl" means C₁-C₆ alkyl-carbonyl.

The term "alkoxy" means alkyloxy, e.g. "C₁-C₆ alkoxy" means C₁-C₆ alkyl-oxy.

In the present context, i.e. in connection with the terms "alkyl", "alkylene", "alkoxy", "acyl", "alkenyl", and "alkynyl", the term "optionally substituted" Is intended to mean that the group in question may be substituted one or several times, preferably 1-3 times, with group(s) selected from hydroxy (which when bound to an unsaturated carbon atom may be present in the tautomeric keto form), C₁-C₆ alkoxy, C₂-C₆ alkenyloxy, carboxy, oxo (forming a keto or aldehyde functionality), C₁-C₆ alkoxycarbonyl, C₁-C₆ acyl, formyl, aryl, aryloxy, arylamino, arylcarbonyl, aryloxycarbonyl, arylcarbonyloxy, arylaminocarbonyl, arylcarbonylamino, heteroaryl, heteroaryloxy, heteroarylamino, heteroarylcarbonyl, heteroaryloxycarbonyl, heteroarylcarbonyloxy, heteroarylaminocarbonyl, heteroarylcarbonylamino, heterocyclyl, heterocyclyloxy, heterocyclylamino, heterocyclylcarbonyl, heterocyclyloxycarbonyl, heterocyclylcarbonyloxy, heterocyclylaminocarbonyl, heterocyclylcarbonylamino, amino, mono- and di(C₁-C₆ alkyl)amino, carbamoyl, mono- and di(C₁-C₆ alkyl)aminocarbonyl, C₁-C₆ acylamino, cyano, guanidino, carbamido, C₁-C₆ alkyl-sulphonyl-amino, aryl-sulphonyl-amlno, heteroaryl-sulphonyl-amino, C₁-C₆ alkanoyloxy, C₁-C₆ alkyl-sulphonyl, C₁-C₆ alkyl-sulphinyl, C₁-C₆ alkylsulphonyloxy, nitro, C₁-C₆ alkylthio, and halogen, where any aryl, heteroaryl and heterocyclyl may be substituted as specifically described below for aryl, heteroaryl and heterocyclyl, and any alkyl, alkoxy, and the like, representing substituents may be substituted with hydroxy, C₁-C₆ alkoxy, amino, mono- and di(C₁-C₆ alkyl)amino, carboxy, C₁-C₆ acylamino, C₁-C₆ alkylaminocarbonyl, or halogen(s).

Typically, the substituents are selected from hydroxy (which when bound to an unsaturated carbon atom may be present in the tautomeric keto form), C₁-C₆ alkoxy, C₂-C₆ alkenyloxy, carboxy, oxo (forming a keto or aldehyde functionality), C₁-C₆ acyl, formyl, aryl, aryloxy, arylamino, arylcarbonyl, heteroaryl, heteroaryloxy, heteroarylamino, heteroarylcarbonyl, heterocyclyl, heterocyclyloxy, heterocyclylamino, heterocyclylcarbonyl, amino, mono- and di(C₁-C₆ alkyl)amino; carbamoyl, mono- and di(C₁-C₆ alkyl)aminocarbonyl, amino-C₁-C₆ alkyl-aminocarbonyl, mono- and di(C₁-C₆ alkyl)amino-C₁-C₆ alkyl-aminocarbonyl, C₁-C₆ acylamino, guanidino, carbamido, C₁-C₆ alkyl-sulphonyl-amino, C₁-C₆ alkyl-sulphonyl, C₁-C₆ alkyl-sulphinyl, C₁-C₆ alkylthio, halogen, where any aryl, heteroaryl and heterocyclyl may be substituted as specifically described below for aryl, heteroaryl and heterocyclyl.

In some embodiments, substituents are selected from hydroxy, C₁-C₆ alkoxy, amino, mono-and di(C₁-C₆ alkyl)amino, carboxy, C₁-C₆ acylamino, C₁-C₆ alkylaminocarbonyl, and halogen.

The term "halogen" includes fluoro, chloro, bromo, and iodo.

The term "Boc" means tert-butoxycarbonyl, i.e. an N-protecting group.

In the present context, the term "aryl" is intended to mean a fully or partially aromatic carbocyclic ring or ring system, such as phenyl, naphthyl, 1,2,3,4-tetrahydronaphthyl, anthracyl, phenanthracyl, pyrenyl, benzopyrenyl, fluorenyl and xanthenyl, among which phenyl is a preferred example.

The term "heteroaryl" is intended to mean a fully or partially aromatic carbocyclic ring or ring system where one or more of the carbon atoms have been replaced with heteroatoms, e.g. nitrogen (=N- or -NH-), sulphur, and/or oxygen atoms. Examples of such heteroaryl groups are oxazolyl, isoxazolyl, thiazolyl, isothiazolyl, pyrrolyl, imidazolyl, pyrazolyl, pyridinyl, pyrimidinyl, pyrazinyl, pyridazinyl, triazinyl, coumaryl, furanyl, thienyl, quinolyl, benzothiazolyl, benzotriazolyl, benzodiazolyl, benzooxozolyl, phthalazinyl, phthalanyl, triazolyl, tetrazolyl, isoquinolyl, acridinyl, carbazolyl, dibenzazepinyl, indolyl, benzopyrazolyl, phenoxazonyl. Particularly interesting heteroaryl groups are benzimidazolyl, oxazolyl, isoxazolyl, thiazolyl, isothiazolyl, pyrrolyl, imidazolyl, pyrazolyl, pyridinyl, pyrimidinyl, pyrazinyl, pyridazinyl, furyl, thienyl, quinolyl, triazolyl, tetrazolyl, isoquinolyl, indolyl in particular benzimidazolyi, pyrrolyl, imidazolyl, pyridinyl, pyrimidinyl, furyl, thienyl, quinolyl, tetrazolyl, and isoquinolyl.

The term "heterocyclyl" is intended to mean a non-aromatic carbocyclic ring or ring system where one or more of the carbon atoms have been replaced with heteroatoms, e.g. nitrogen (=N- or -NH-), sulphur, and/or oxygen atoms. Examples of such heterocyclyl groups (named according to the rings) are imidazoildine, piperazine, hexahydropyridazine, hexahydropyrimidine, diazepane, diazocane, pyrrolidine, piperidine, azepane, azocane, aziridine, azirine, azetidine, pyroline, tropane, oxazinane (morpholine), azepine, dihydroazepine, tetrahydroazepine, and hexahydroazepine, oxazolane, oxazepane, oxazocane, thiazolane, thiazinane, thiazepane, thiazocane, oxazetane, diazetane, thiazetane, tetrahydrofuran, tetrahydropyran, oxepane, tetrahydrothiophene, tetrahydrothiopyrane, thiepane, dithiane, dithiepane, dioxane, dioxepane, oxathiane, oxathiepane. The most interesting examples are tetrahydrofuran, imidazolidine, piperazine, hexahydropyridazine, hexahydropyrimidine, diazepane, diazocane, pyrrolidine, piperidine, azepane, azocane, azetidine, tropane, oxazinane (morpholine), oxazolane, oxazepane, thiazolane, thiazinane, and thiazepane, in particular tetrahydrofuran, imidazolidine, piperazine, hexahydropyridazine, hexahydropyrimidine, diazepane, pyrrolidine, piperidine, azepane, oxazinane (morpholine), and thiazinane.

The expression "4- to 8-membered heterocyclic ring" is intended to means a ring of the type specified above under "heteroaryl" and "heterocyclyl" provided that the ring comprises 4 to 8 ring atoms.

In the present context, *i.e*. in connection with the terms "aryl", "heteroaryl", "heterocyclyl", "4- to 8-membered heterocyclic ring", and the like (e.g. "aryloxy", "heterarylcarbonyl", etc.), the term "optionally substituted" is intended to mean that the group in question may be substituted one or several times, preferably 1-5 times, in particular 1-3 times, with group(s) selected from hydroxy (which when present in an enol system may be represented in the tautomeric keto form), C₁-C₆ alkyl, C₁-C₆ alkoxy, C₂-C₆ alkenyloxy, oxo (which may be represented in the tautomeric enol form), carboxy, C₁-C₆ alkoxycarbonyl, C₁-C₆ acyl, formyl, aryl, aryloxy, arylamino, aryloxycarbonyl, arylcarbonyl, heteroaryl, heteroarylamino, amino, mono- and di(C₁-C₆ alkyl)amino; carbamoyl, mono- and di(C₁-C6 alkyl)aminocarbonyl, amino-C₁-C₆ alkyl-aminocarbonyl, mono- and di(C₁-C₆ alkyl)amino-C₁-C₆ alkyl-aminocarbonyl, C₁-C₆ acylamino (e.g. acetamido), cyano, guanidino, carbamido, C₁-C₆ alkanoyloxy, C₁-C₆ alkyl-sulphonyl-amino, aryl-sulphonyl-amino, heteroaryl-sulphonyl-amino, C₁-C₆ alkyl-suphonyl, C₁-C₆ alkyl-sulphinyl, C₁-C₆ alkylsulphonyloxy, nitro, sulphanyl, amino, amino-sulfonyl, mono-and di(C₁-C₆ alkyl)amino-sulfonyl, hatogen-C₁-C₄ alkyl, dihalogen-C₁-C₄ alkyl, trihalogen-C₁-C₄ alkyl, halogen, where aryl and heteroaryl representing substituents may be substituted 1-3 times with C₁-C₄ alkyl, C₁-C₄ alkoxy, nitro, cyano, amino or halogen, and any alkyl, alkoxy; and the like, representing substituents may be substituted with hydroxy, C₁-C₆ alkoxy, C₂-C₆ alkenyloxy, amino, mono- and di(C₁-C₆ alkyl)amino, carboxy, C₁-C₆ acylamino, halogen, C₁-C₆ alkylthio, C₁-C₆ alkyl-sulphonyl-amino, or guanidino.

Typically, the substituents are selected from hydroxy, C₁-C₆ alkyl, C₁-C₆ alkoxy, oxo (which may be represented In the tautomeric enol form), carboxy, C₁-C₆ acyl, formyl, amino, mono-and di(C₁-C₆ alkyl)amino; carbamoyl, mono- and di(C₁-C₆ alkyl)aminocarbonyl, amino-C₁-C₆ alkyl-aminocarbonyl, C₁-C₆ acylamino, guanidino, carbamido; C₁-C₆ alkyl-sulphonyl-amino, aryl-sulphonyl-amino, heteroaryl-sulphonyl-amino, C₁-C₆ alkyl-suphonyl, C₁-C₆ alkyl-sulphinyl, C₁-C₆ alkylsulphonyloxy, sulphanyl, amino, amino-sulfonyl, mono- and di(C₁-C₆ alkyl)amino-sulfonyl or halogen, where any alkyl, alkoxy and the like, representing substituents may be substituted with hydroxy, C₁-C₆ alkoxy, C₂-C₆ alkenyloxy, amino, mono-and di(C₁-C₆ alkyl)amino, carboxy, C₁-C₆ acylamino, halogen, C₁-C₆ alkylthio, C₁-C₆ alkyl-sulphonyl-amino, or guanidino. In some embodiments, the substituents are selected from C₁-C₆ alkyl, C₁-C₆ alkoxy, amino, mono- and di(C₁-C₆ alkyl)amino, sulphanyl, carboxy or halogen, where any alkyl, alkoxy and the like, representing substituents may be substituted with hydroxy, C₁-C₆ alkoxy, C₂-C₆ alkenyloxy, amino, mono- and di(C₁-C₆ alkyl)amino, carboxy, C₁-C₆ acylamino, halogen, C₁-C₆ alkylthio, C₁-C₆ alkyl-sulphonyl-amino, or guanidino.

The term "pharmaceutically acceptable salts" is intended to include acid addition salts and basic salts. Illustrative examples of acid addition salts are pharmaceutically acceptable salts formed with non-toxic acids. Exemplary of such organic salts are those with maleic, fumaric, benzoic, ascorbic, succinic, oxalic, bis-methylenesalicyllc, methanesulfonlc, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic, and theophylline acetic acids, as well as the 8-halotheophyllines, for example 8-bromotheophylline. Exemplary of such inorganic salts are those with hydrochloric, hydrobromic, sulfuric, sulfamic, phosphoric, and nitric acids. Examples of basic salts are salts where the (remaining) counter ion is selected from alkali metals, such as sodium and potassium, alkaline earth metals, such as calcium, and ammonium ions (⁺N(R)₃R', where R and R' independently designates optionally substituted C₁-C₆ alkyl, optionally substituted C₂-C₆ alkenyl, optionally substituted aryl, or optionally substituted heteroaryl). Pharmaceutically acceptable salts and pharmaceutically acceptable carrierss are, e.g., those described In Remington's Pharmaceutical Sciences, 17. Ed. Alfonso R. Gennaro (Ed.), Mack Publishing Company, Easton, PA, U.S.A., 1985 and more recent editions and In Encyclopedia of Pharmaceutical Technology. Thus, the term "an acid addition salt or a basic salt thereof" used herein is intended to comprise such salts. Furthermore, the compounds as well as any intermediates or starting materials may also be present in hydrate form.

### Compounds of the Formula I

B Is typically a natural or non natural nucleobase selected form the group consisting of adenine, cytosine, 5-methylcytosine, isocytosine, pseudoisocytosine, guanine, thymine, uracil, 5-bromouracil, 5-propynyluracil, 5-propyny-6-fluorouracil, 5-methylthiazoteuracil, 6-aminopurine, 2-aminopurine, inosine, diaminopurine, 7-propyne-7-deazaadenine, 7-propyne-7-deazaguanine, and 2-chloro-6-aminopurine.

In one embodiment B is adenine or an adenine analogue.

In one embodiment B is cytosine or a cytosine analogue.

In one embodiment B is guanine or a guanine analogue.

In one embodiment B is thymine or a thymine analogue.

In one embodiment B is selected from the group consisting of adenine, cytosine and guanine, and analogues thereof.

X in Formula I is selected from the group consisting of -O-, -S-, >NH and >NR', wherein R' is selected from the group consisting of hydrogen, C₁-C₆acyl (e.g. acetyl and benzyl) and C₁-C₆ alkyl (e.g. methyl and isopropyl).

Compounds of Formula I may exist as depicted below 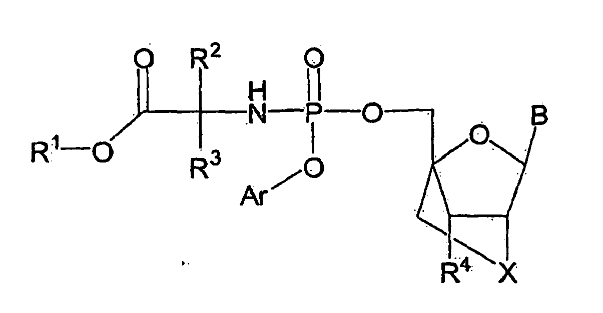 the β-D form

The compounds of the invention are in the β -D form. In β -D formation, preferably X is either -O- (β -D-oxy).

Ar is a substituted or an unsubstituted aryl group

In one embodiment, Ar is substituted or an unsubstituted monocyclic aromatic ring moiety or a fused bicyclic aromatic ring moiety, preferably a substituted or an unsubstituted phenyl group,

Therefore, Ar may be a substituted monocyclic aromatic ring moiety or a fused bicyclic aromatic ring moiety, wherein said substitution is selected from the group consisting of halogen (preferably chlorine or fluorine), trihalomethyl (preferably trifiuoromethyl), cyano and nitro groups. A preferable substituent is a halogen, such as chlorine or fluorine.

In one embodiment, R¹ is selected from the group consisting of methyl, ethyl, n-or I-propyl, n-or i-butyl, or benzyl. In a preferable embodiment, R¹ is selected from the group consisting of -Me and -Bn, preferably benzyl (-Bn)

In one embodiment R² and R³ are, independently selected from the group consisting of Hydrogen, methyl, secondary butyl, benzyl, or, together with the C atom to which they are attached, provide a C₅-C₆ ring.

In one embodiment, R² Is -H or Me (hydrogen or methyl).

In one embodiment, R³ is -H or Me.

In the same or different embodiments, R⁴ is R⁴ is selected from the group consisting of hydrogen, hydroxyl, amino, azido, and halo;

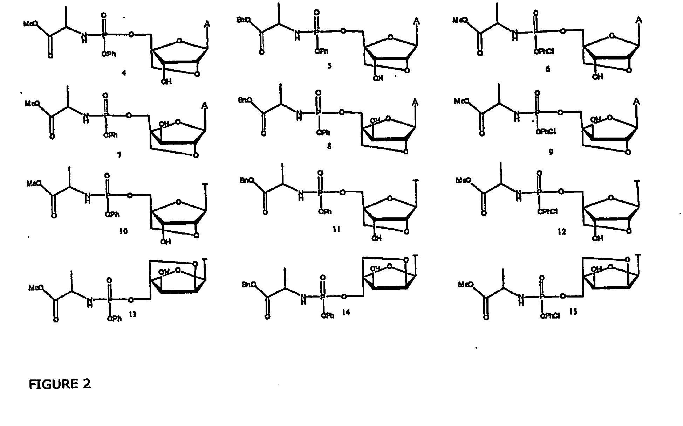

Compounds of the invention, or for use in the composition of the invention include the list shown in figure 2.

Methods for the synthesis of the above compounds are provided herein (see "Synthesis of Compounds" further below).

Preferred compounds include one or more of the following: 4, 5, and 6. (see Figure 2)

Most preferred compounds may include: 5 and 6.

The compounds of Formula I, such as those used within the compositions and or methods of the present invention, may be selected from the group comprising a β-D-oxy, a xylo or an alpha-L-oxy locked nucleoside.

### Pharmaceutical compositions

The compounds may suitable be formulated as a pharmaceutical composition in order to facilitate the proper absorbance of the compound in the relevant region or tissue.

Hence, the invention also provides a pharmaceutical composition comprising compound of Formula I (as defined herein) and a pharmaceutically acceptable carrier.

### Medical use of compounds of Formula I

According to a further aspect of the present invention there is provided a compound having formula I according to the present invention for use in a method of treatment, preferably In the prophylaxis or treatment of cancer.

There is disclosed a method of phrophylaxis or treatment of cancer comprising administration to a patient in need of such treatment an effective dose of a compound having formula I according to the present invention.

According to a further aspect of the present invention there is provided use of a compound having formula I of the present invention in the manufacture of a medicament for use in the treatment or prophlylaxis of cancer.

According to a further aspect of the present invention there is provided a pharmaceutical composition comprising a compound having formula I of the present invention in combination with a pharmaceutically acceptable excipient, carrier or diluent.

According to a further aspect of the present invention there is provided a method of preparing a pharmaceutical composition comprising the step of combining a compound having formula I of the present invention with a pharmaceutically acceptable excipient, carrier or diluent.

In one embodiment, the pharmaceutical composition of the invention further comprises anti-inflamatory compounds and/or antiviral compounds.

In one embodiment, the pharmaceutical composition of the invention comprises two or more non-identical compounds of Formula I, for example the pharmaceutical composition according to the invention may comprise both compound 5 and compound 6, or compounds 4 and 6, or compounds 4 and 5, or compounds 4, 5 and 6 (see Figure 2).

Disclosed is a method of preventing or treating cancer comprising a therapeutically effective amount of the compound of the invention.

The cancer, which may be treated by the pharmaceutical composition according to the invention, may be selected form the group consisting of: non-Hodgkin's lymphoma, Hodgkin's lymphoma, leukemia (e.g., acute leukemia such as acute lymphocytic leukemia, acute myelocytic leukemia, chronic myeloid leukemia, chronic lymphocytic leukemia, multiple myeloma), colon carcinoma, rectal carcinoma, pancreatic cancer, breast cancer, ovarian cancer, prostate cancer, renal cell carcinoma, hepatoma, bile duct carcinoma, choriocarcinoma, cervical cancer, testicular cancer, lung carcinoma, bladder carcinoma, melanoma, head and neck cancer, brain cancer, cancers of unknown primary site, neoplasms, cancers of the peripheral nervous system, cancers of the central nervous system, tumors (e.g., fibrosarcoma, myxosarcoma, liposarcoma, chondrosarcoma, osteogenic sarcoma, chordoma, angiosarcoma, endothellosarcoma, lymphanglosarcoma, lymphangloendothellosarcoma, synovioma, mesothelioma, Ewing's tumor, lelomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, seminoma, embryonal carcinoma, Wilms' tumor, small cell lung carcinoma, epithelial carcinoma, glioma, astrocytoma, medulloblastoma, craniopharyngioma, ependymoma, pinealoma, hemangloblastoma, acoustic neuroma, oligodendroglioma, meningioma, neuroblastoma, and retinoblastoma), heavy chain disease, metastases, and any disease or disorder characterized by uncontrolled or abnormal cell growth.

In one embodiment, the term 'treatment' as used herein refers to both treatment of an exisiting disease (e.g. a cancer as herein referred to), or prevention of a disease, i.e. prophylaxis.

The invention also provides for the use of the compound or conjugate of the invention as described for the manufacture of a medicament for the treatment of cancer. Preferably said medicament is for the treatment of cancer which is in the form of i) a solid tumor and/or a carcinoma, and/or ii) a sarcoma, and/or iii) glioma.

Preferably said carcinoma as referred to in the use(s) according to the invention, such as use for the manufacture of a medicament and/ or methods according to the invention, such as methods for treating cancer, is selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumors. More preferably, said carcinoma is selected from the group consisting of malignant melanoma, non-small cell lung cancer, breast carcinoma, colon carcinoma and renal cell carcinoma. Preferably, said carcinoma is a malignant melanoma, preferably selected from the group consisting of superficial spreading melanoma, nodular melanoma, lentigo maligna melanoma, acral melagnoma, amelanotic melanoma and desmoplastic melanoma.

Preferably said sarcoma as referred to in the use(s), such as use for the manufacture of a medicament and/or methods according to the invention, such as methods for treating cancer, is selected from the group consisting of osteosarcoma, Ewing's sarcoma, chondrosarcoma, malignant fibrous histiocytoma, fibrosarcoma and Kaposi's sarcoma.

Disclosed is also a method for treating cancer, said method comprising administering a compound according to the invention as herein described, and/or a conjugate according to the invention, and/or a pharmaceutical composition according to the invention to a patient in need thereof.

Preferably, the cancer referred to in the methods of treatment according to the invention and/or uses for the manufacture of a medicament, are selected from the group consisting of cancer diseases is a lung, breast, colon, prostate, pancreas, lung, liver, thyroid, kidney, brain, testes, stomach, intestine, bowel, spinal cord, sinuses, bladder, urinary tract or ovaries cancer.

The invention provides for the use of the compounds per se or as conjugate as herein defined for the manufacture of a medicament for the treatment of cancer, and/or for reducing inflammation associated with the cancers herein disclosed, preferably lung cancer.

### EXAMPLES

The phosphorodichloridates (**1**) and phosphochloridates (**2**) were synthesized according to McGuigan et al (references 1 and 2).

The LNA nucleosides were synthesized according to Koshkin et al (reference 3).

### Standard procedure for the synthesis of phosphoroamldate derivatives:

The nucleoside (1 mol eq.) was evaporated twice from anhydrous pyridine and dried over P₂O₅ for 2 h then dissolved in pyridine/acetonitrile (2:1; 10 mL/ mol. Eq.). To this solution was added ^{t}BuMgCl (1M in THF; 1.1 mol eq) and the solution was stirred at room temperature under argon for 15 min. The appropriate phosphochloridate (0.2M in THF, 2 mol eq.) was added and the solution was stirred under argon for 24 h.

In some cases it was necessary to add further ^{t}BuMgCl (1M in THF, 2 mol eq.) and the appropriate phosphochloridate (0.2M in THF, 2 mol eq.). After another 24h the solution was concentrated in vacuo and redissolved in DCM (50 mL/ mol eq.). The DCM solution was washed with HCl (1N, 50 mL/ mol eq) and brine (2 x 50 mL/ mol eq). The combined organic phase was dried with MgSO₄ and concentrated in vacuo. Purified by DCVC (reference 4), eluted with MeOH in DCM 0-10 %.

### EXAMPLE 1: Synthesis of Phenyl-[Benzyloxy-L-alaninyl]-[((1R,3R,4R,7S)-3-(adenine-9-yl)-7-hydroxy-2,5-dioxablcyclo[2:2:1]heptane-1-yl)methyl] phosphate (5):

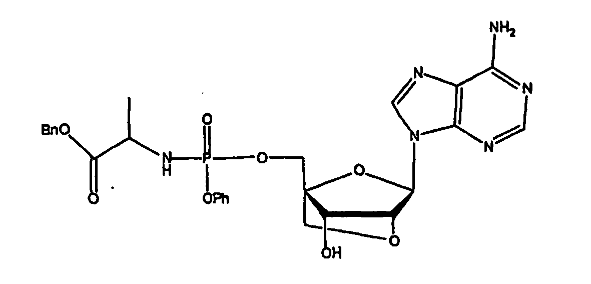

(1*S*,3*R*,4*R*,7*S*)-3-(adenine-9-yl)-7-hydroxy-1-hydroxymethyl-2,5-dioxabicyclo[2:2:1]heptane (125 mg, 0,45mmol) was evaporated twice from anhydrous pyridine and dried over P₂O₅ for 2 h. hereafter it was dissolved in pyridine/acetonitrile (2:1; 5 mL). To this solution was added ^{t}BuMgCl (1M in THF, 0.5 mL, 0.5mmol) and the solution was stirred at room temperate under argon for 15 min. The Phenyl-[Benzyloxy-L-alaninyl]phosphorchloridate (0.2M in THF, 4.5 mL, 0.9 mmol) was added and the solution was stirred under argon for 24 h. After this time ^{t}BuMgCl (1M In THF, 0.5 ml, 0.5mmol) was added followed by the The Phenyl-[Benzyloxy-L-alaninyl]phosphorchloridate (0.2M in THF, 4.5 mL, 0.9mmol). After another 24h the solution was concentrated in vacuo and redissolved in DCM (25 mL). The DCM solution was washed with HCl (1N, 25mL) and brine (2 x 25 mL). Hereafter It was dried with MgSO₄ and conc. in vacuo. Purified by DCVC⁴, eluted with MeOH in DCM 0-10 %. Purified yield: 13 mg
MS(ESI) Found [M+H]⁺ 597.2
³¹P NMR (400 MHz, CDCl₃) δₚ: 4.53 (double peak)
¹H NMR (400 MHz, DMSO-d₆) δₚ: 8.21 (1H, s, H2); 8.15 (1H, s, H8); 7.39-7.11 (10H, m, 2 x Ph); 6.12 (1H, m, CHCH₃); 5.91 (1H, s, H'1); 5.06 (2H, d, J=5.35 Hz); 4.48-4.32 (4H, m, H'5 + Ph-CH₂); 3.94 (1H, d, ³J^{HH}=8,05, H'2/H'3); 3.75 (1H, d, ³j^{HH} =8.00, H'2/H'3); 1.27 (3H, d, ³J^{HH}=7.02Hz, CH3)

### EXAMPLE 2: Synthesis of Phenyl-[Methoxy-L-alaninyl]-[((1R,3R,4R,7S)-3-(adenine-9-yl)-7-hydroxy-2,5-dioxabicyclo[2:2:1]heptane-1-yl)methyl] phosphate (4)

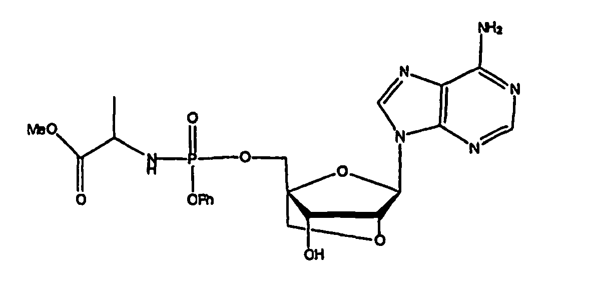

The synthesis of **4** was done by using Phenol, Methoxy-L-alanine hydrochloride and (1*S*,3*R*,4*R*,7*S*)-3-(adenine-9-yl)-7-hydroxy-1-hydroxymethyl-2,5-dioxabicyclo[2:2:1]heptane as starting materials for the standard produces.
MS(ESI) Found [M+H]⁺ 521.2
³¹P NMR (400 MHz, CDCl₃) δₚ: 5.37; 4.35

### EXAMPLE 3: Synthesis of Phenyl-[Benzyloxy-L-alaninyl]-[((1R,3R,4R,7S)-3-(adenine-9-yl)-7-hydroxy-2,5-dioxabicyclo[2:2:1]heptane-1-yl)methyl] phosphate (5):

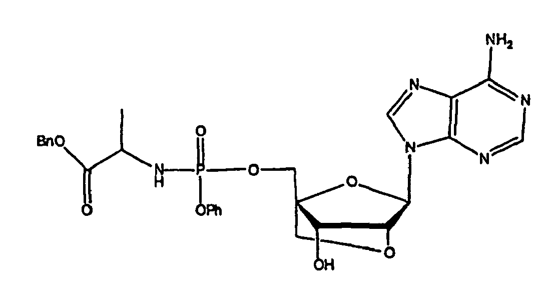

The synthesis of **5** was done by using Phenol, Benzyloxy-L-alanine hydrochloride and (1*S*,3*R*,4*R*,7*S*)-3-(adenine-9-yl)-7-hydroxy-1-hydroxymethyl-2,5-dioxabicyclo[2:2:1]heptane as starting materials for the standard produces.
MS(ESI) Found [M+H]⁺ 597.2
³¹P NMR (400 MHz, CDCl₃) δₚ: 5.04; 4.46
¹H NMR (400 MHz, DMSO-d₆) δₚ: 8.21 (1H, s, H2); 8.15 (1H, s, H8); 7.39-7.11 (10H, m, 2 x Ph); 6.12 (1H, m, CHCH₃); 5.91 (1H, s, H'1); 5.06 (2H, d, J=5.35 Hz); 4.48-4.32 (4H, m, H'5 + Ph-CH₂); 3.94 (1H, d, ³J^{HH}=8,05, H'2/H'3); 3.75 (1H, d, ³J^{HH}=8.00, H'2/H'3); 1.27 (3H, d, ³J^{HH}=7.02Hz, CH3)

### EXAMPLE 4: Synthesis of p-chloro-phenyl-[Benzyloxy-L-alaninyl]-[((1R,3R,4R,7S)-3-(adenine-9-yl)-7-hydroxy-2,5-dioxabicyclo[2:2:1]heptane-1-yl)methyl] phosphate (6):

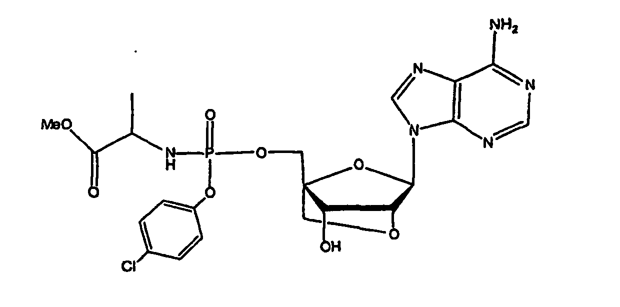

The synthesis of **6** was done by using p-chlorophenol, Methoxy-L-alanine hydrochloride and (1*S*,3*R*,4*R*,7*S*)-3-(adenine-9-yl)-7-hydroxy-1-hydroxymethyl-2,5-dioxabicyclo[2:2:1]heptane as starting materials for the standard produces.
MS(ESI) Found [M+H]⁺ 555.0

### EXAMPLE 5: Synthesis of Phenyl-[Methoxy-L-alaninyl]-[((1R,3R,4R,7R)-3-(adenine-9-yl)-7-hydroxy-2,5-dioxabicyclo[2:2:1]heptane-1-yl)methyl] phosphate (7)

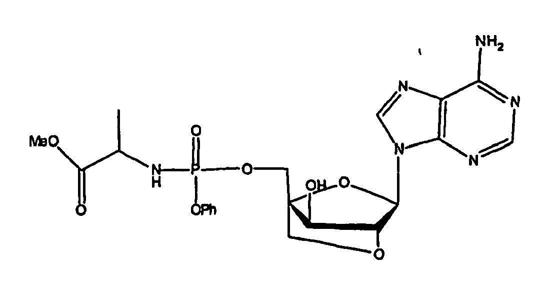

The synthesis of **7** was done by using Phenol, Methoxy-L-alanine hydrochloride and (1*S*,3*R*,4*R*,7*R*)-3-(adenine-9-yl)-7-hydroxy-1-hydroxymethyl-2,5-dioxablcyclo[2:2:1]heptane as starting materials for the standard produces.
MS(ESI) Found [M+H]⁺ 521.1
³¹P NMR (400 MHz, CDCl₃) δₚ: 5.95; 5.29

### EXAMPLE 6: Synthesis of Phenyl-[Benzyloxy-L-alaninyl]-[((1R,3R,4R,7R)-3-(adenine-9-yl)-7-hydroxy-2,5-dioxabicyclo[2:2:1]heptane-1-yl)methyl] phosphate (8):

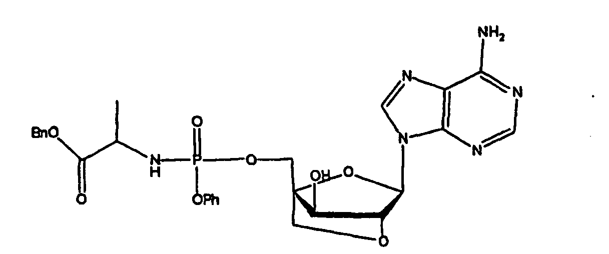

The synthesis of **8** was done by using Phenol, Benzyloxy-L-alanine hydrochloride and (1*S*,3*R*,4*R*,7*R*)-3-(adenine-9-yl)-7-hydroxy-1-hydroxymethyl-2,5-dioxabicyclo[2:2:1]heptane as starting materials for the standard produces.
MS(ESI) Found [M+H]⁺ 597.2
³¹P NMR (400 MHz, CDCl₃) δₚ: 5.11; 4.46

### EXAMPLE 7: Synthesis of p-chloro-phenyl-[Benzyloxy-L-alaninyl]-[((1R,3R,4R,7R)-3-(adenine-9-yl)-7-hydroxy-2,5-dioxabicyclo[2:2:1]heptane-1-yl)methyl] phosphate (9):

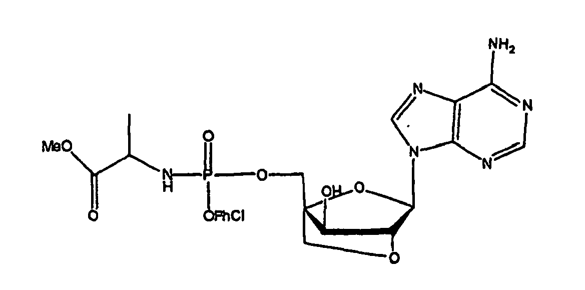

The synthesis of **9** was done by using p-chlorophenol, Methoxy-L-alanine hydrochloride and (1*S*,3*R*,4*R*,7*R*)-3-(adenine-9-yl)-7-hydroxy-1-hydroxymethyl-2,5-dioxabicyclo[2:2:1]heptane as starting materials for the standard produces.
MS(ESI) Found [M+H]⁺ 555.0

### EXAMPLE 8: Synthesis of Phenyl-[Methoxy-L-alaninyl]-[((1R,3R,4R,7R)-3-(adenine-9-yl)-7-hydroxy-2,5-dioxabicyclo[2:2:1]heptane-1-yl)methyl] phosphate (7)

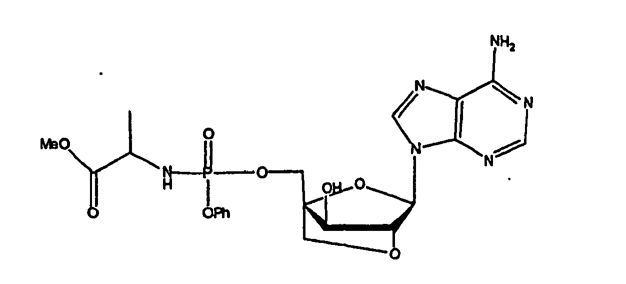

The synthesis of **7** was done by using Phenol, Methoxy-L-alanine hydrochloride and (1*S*,3*R*,4*R*,7*R*)-3-(adenine-9-yl)-7-hydroxy-1-hydroxymethyl-2,5-dioxabicyclo[2:2:1]heptane as starting materials for the standard produces.
MS(ESI) Found [M+H]⁺ 521.1
³¹P NMR (400 MHz, CDCl₃) δₚ: 5.95; 5.29

### EXAMPLE 9: Synthesis of Phenyl-[Benzyloxy-L-alaninyl]-[((1R,3R,4R,7R)-3-(adenine-9-yl)-7-hydroxy-2,5-dioxabicyclo[2:2:1]heptane-1-yl)methyl] phosphate (8):

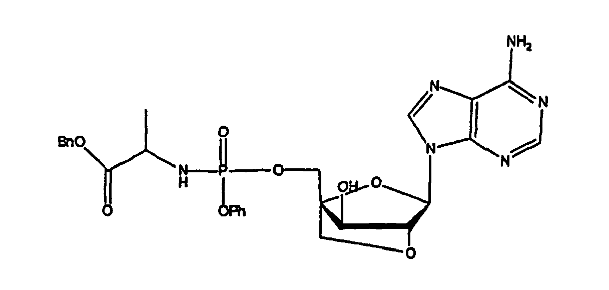

The synthesis of **8** was done by using Phenol, Benzyloxy-L-alanine hydrochloride and (1*S*,3*R*,4*R*,7*R*)-3-(adenine-9-yl)-7-hydroxy-1-hydroxymethyl-2,5-dioxabicyclo[2:2:1]heptane as starting materials for the standard produces.
MS(ESI) Found [M+H]⁺ 597.2
³¹P NMR (400 MHz, CDCl₃) δₚ: 5.11; 4.46

### EXAMPLE 10: Synthesis of p-chloro-phenyl-[Benzyloxy-L-alaninyl]-[((1R,3R,4R,7R)-3-(adenine-9-yl)-7-hydroxy-2,5-dioxabicyclo[2:2:1]heptane-1-yl)methyl] phosphate (9):

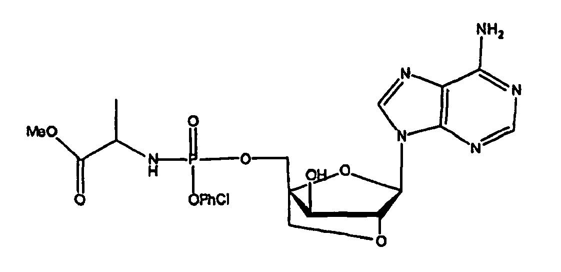

The synthesis of **9** was done by using p-chlorophenol, Methoxy-L-alanine hydrochloride and (1*S*,3*R*,4*R*,7*R*)-3-(adenine-9-yl)-7-hydroxy-1-hydroxymethyl-2,5-dioxabicyclo[2:2:1]heptane as starting materials for the standard produces.
MS(ESI) Found [M+H]⁺ 555.0

### EXAMPLE 11: Synthesis of Phenyl-[Methoxy-L-alaninyl]-[((1R,3R,4R,7S)-3-(thymin-1-yl)-7-hydroxy-2,5-dioxabicyclo[2:2:1]heptane-1-yl)methyl] phosphate (10)

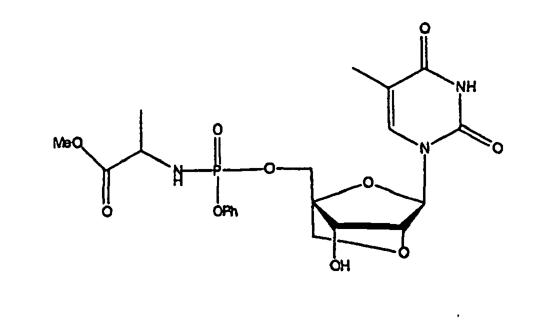

The synthesis of **10** was done by using Phenol, Methoxy-L-alanine hydrochloride and (1*S*,3*R*,4*R*,7*S*)-3-(thymin-1-yl)-7-hydroxy-1-hydroxymethyl-2,5-dioxabicyclo[2:2:1]heptane as starting materials for the standard produces.
MS(ESI) Found [M+H]⁺ 512.1
³¹P NMR (400 MHz, COCl₃) δₚ: 4.93; 4.78

### EXAMPLE 12: Synthesis of Phenyl-[Benzyloxy-L-alaninyl]-[((1R,3R,4R,7S)-3-(thymin-1-yl)-7-hydroxy-2,5-dioxabicyclo[2:2:1]heptane-1-yl)methyl] phosphate (11):

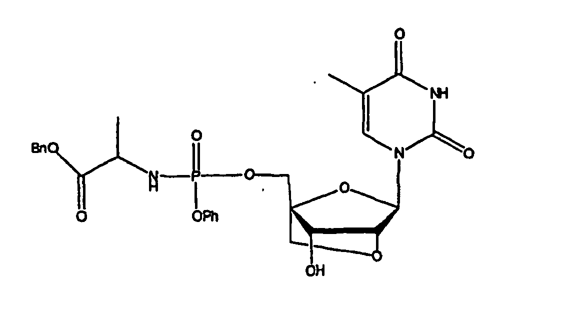

The synthesis of **11** was done by using Phenol, Benzyloxy-L-alanine hydrochloride and (1*S*,3*R*,4*R*,7*S*)-3-(thymin-1-yl)-7-hydroxy-1-hydroxymethyl-2,5-dioxabicyclo[2:2:1]heptane as starting materials for the standard produces.
MS(ESI) Found [M+H]⁺ 588.2
³¹P NMR (400 MHz, CDCl₃) δₚ: 5.75;4.19

### EXAMPLE 13: Synthesis of p-chloro-phenyl-[Benzyloxy-L-alaninyl]-[((1R,3R,4R,7S)-3-(thymin-1-yl)-7-hydroxy-2,5-dioxabicyclo[2:2:1]heptane-1-yl)methyl] phosphate (12):

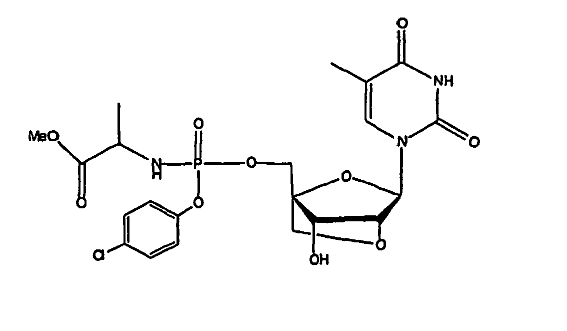

The synthesis of **12** was done by using p-chlorophenol, Methoxy-L-alanine hydrochloride and (1*S*,3*R*,4*R*,7*S*)-3-(thymln-1-yl)-7-hydroxy-1-hydroxymethyl-2,5-dioxabicyclo[2:2:1]heptane as starting materials for the standard produces.
MS(ESI) Found [M+H]⁺ 546.0
³¹P NMR (400 MHz, CDCl₃) δₚ: 5.04; 4.46

### EXAMPLE 14: Synthesis of Phenyl-[Methoxy-L-alaninyl]-[((1S,3R,4S,7R)-3-(thymin-1-yl)-7-hydroxy-2,5-dioxabicyclo[2:2:1]heptane-1-yl)methyl] phosphate (13)

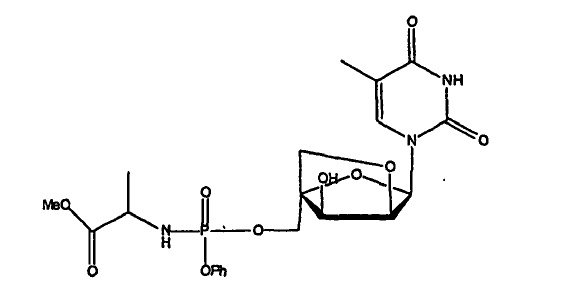

The synthesis of **13** was done by using Phenol, Methoxy-L-alanine hydrochloride and (1*R*,3*R*,4*S*,7*R*)-3-(thymin-1-yl)-7-hydroxy-1-hydroxymethyl-2,5-dioxabicyclo[2:2:1]heptane as starting materials for the standard produces.
MS(ESI) Found [M+H]⁺ 512.1
³¹P NMR (400 MHz, CDCl₃) δₚ: 5.43; 4.86

### EXAMPLE 15: Synthesis of Phenyl-[Benzyloxy-L-alaninyl]-[((1S,3R,4S,7R)-3-(thymin-1-yl)-7-hydroxy-2,5-dioxabicyclo[2:2:1]heptane-1-yl)methyl] phosphate (14):

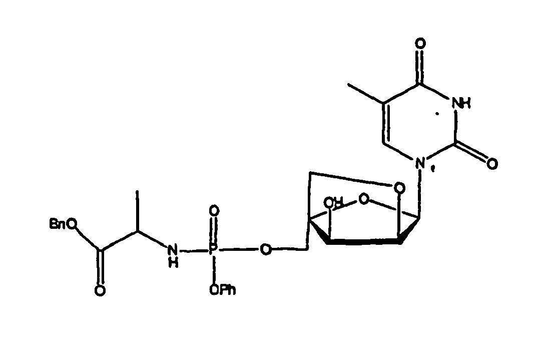

The synthesis of **14** was done by using Phenol, Benzyloxy-L-alanine hydrochloride and (1*R*,3*R*,4*S*,7*R*)-3-(thymin-1-yl)-7-hydroxy-1-hydroxymethyl-2,5-dioxabicyclo[2:2:1]heptane as starting materials for the standard produces.
MS(ESI) Found [M+H]⁺ 588.2
³¹P NMR (400 MHz, CDCl₃) δₚ: 5.30; 4.64

### EXAMPLE 16: Synthesis of p-chloro-phenyl-[Benzyloxy-L-alaninyl]-[((1S,3R,4S,7R)-3-(thymin-1-yl)-7-hydroxy-2,5-dioxabicyclo[2:2:1]heptane-1-yl)methyl] phosphate (15):

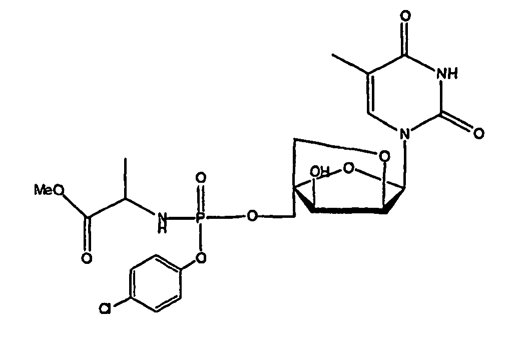

The synthesis of **15** was done by using p-chlorophenol, Methoxy-L-alanine hydrochloride and (1*R*,3*R*,4*S*,7*R*)-3-(thymin-1-yl)-7-hydroxy-1-hydroxymethyl-2,5-dioxabicyclo[2:2:1]heptane as starting materials for the standard produces.
MS(ESI) Found [M+H]⁺ 546.0
³¹P NMR (400 MHz, CDCl₃) δₚ: 4.66; 4.40

### EXAMPLE 17: MTS assay

15PC3 cells were seeded to a density of 12000 cells per well in white 96 well plate (Nunc 136101) in DMEM the day prior to transfection. The next day, cells were washed once in prewarmed OptiMEM followed by addition of 72 µl OptiMEM containing 5 µg/ml Lipofectamine2000 (In vitrogen). Cells were incubated for 7 min. before adding 18 µl LNA phosphoramidate diluted in OptiMEM. The final LNA phosphoramidate concentration ranged from 0,1 nM to 500 nM. After 4 h of treatment, cells were washed in OptiMEM and 100 µl serum containing DMEM was added. Following treatment with the LNA phosphoramidate compound, cells were allowed to recover for the period indicated, viable cells were measured by adding 20 µl the tetrazolium compound [3-(4,5-dimethyl-2-yl)-5-(3-carboxymethoxyphenyl)-2-(4-sulfophenyl)-2H-tetrazolium, inner salt; MTS] and an electron coupling reagent (phenazine ethosulfate; PES) (CellTiter 96^{®} AQᵤₑₒᵤₛ One Solution Cell Proliferation Assay, Promega) per 100 µl DMEM. Viable cells were measured at 490 nm in a Powerwave (Biotek Instruments). Growth rates (δOD/h) were plotted against the concentration of the LNA phosphoramidate.

### Reference List

(1) McGuigan, C.; Thiery, J. C.; Daverio, F.; Jiang, W. G.; Davies, G.; Mason, M. Bioorg.Med.Chem. 2005, 13, 3219-3227.
(2) McGuigan, C. Chemical compounds. WO 2005/012327 A2. 20-7-2004.
(3) Koshkin, A. A.; Fensholdt, J.; Pfundheller, H. M.; Lomholt, C. J.org.chem. 2001, 66, 8504-8512.
(4) Pedersen, D. S.; Rosenbohm, C. Synthesis 2001, 2431-2434.

## Claims

1. A compound of formula I for use as a medicament 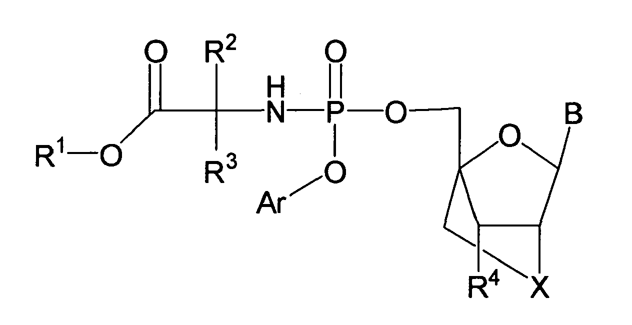 Formula I
wherein:
B is a nucleobase;
Ar is a substituted or unsubstituted aryl group;
X is selected from the group consisting of -O-, -S-, >NH and >NR', wherein R' is selected from the group consisting of hydrogen, C₁-C₆ acyl, C₁-C₆ alkyl and C₃-C₆ cycloalkyl;
R¹ is selected from the group consisting alkyl, cycloalkyl, aryl and alkylaryl;
R² and R³ are, independently, selected from the group comprising H, C₁₋₆ primary, secondary or tertiary alkyl, C₁-C₃ alkyl, C₅-C₇ aryl, C₃-C₆ cycloalkyl, or, when together they form an alkylene chain, they provide, together with the C atom to which they are attached, a C₃-C₈ carbocyclic aliphatic ring;
R⁴ is selected from the group consisting of hydrogen, hydroxyl, amino, azido, halo, optionally substituted C₁-C₆ alkyl, optionally substituted C₃-C₆ cycloalkyl, optionally substituted C₁-C₆ alkoxy, carboxy, nitrilo, nitro, aryl, thiol, and -Y-CO-Rd, wherein Y is selected from the group consisting of O, >NH and -S , and Rd is selected from the group consisting of NH₂, OH, C₁-C₆ alkyl and C₃-C₆ cycloalkyl,
and , wherein B is a natural or non natural nucleobase selected form the group consisting of adenine, cytosine, 5-methylcytosine, isocytosine, pseudoisocytosine, guanine, thymine, uracil, 5-bromouracil, 5-propynyluracil, 5-propyny-6-fluorouracil, 5-methylthiazoleuracil, 6-aminopurine, 2-aminopurine, inosine, diaminopurine, 7-propyne-7-deazaadenine, 7-propyne-7-deazaguanine, and 2-chloro-6-aminopurine.

2. The compound for use as a medicament according to claim 1, wherein B is adenine or an adenine analogue.

3. The compound for use as a medicament according to claim 1 or 2 , wherein Ar is substituted or unsubstituted monocyclic aromatic ring moiety, such as a substituted or unsubstituted phenyl group, or a fused bicyclic aromatic ring moiety.

4. The compound for use as a medicament according to claim 3, wherein Ar is a substituted monocyclic aromatic ring moiety or a fused bicyclic aromatic ring moiety, wherein said substitution is selected from the group consisting of halogen, trihalomethyl, cyano and nitro groups.

5. The compound for use as a medicament according to claim 4, wherein the substituent is a halogen, such as chlorine or fluorine.

6. The compound for use as a medicament according to any one of claims 1 - 5, wherein X is selected from -O-, -S-, and -NH-.

7. The compound for use as a medicament according to any one of claims 1 - 6, wherein R¹ is selected from the group consisting of methyl, ethyl, n-or i-propyl, n-or i-butyl, or benzyl.

8. The compound for use as a medicament according to claim 7, wherein R¹ is methyl or benzyl.

9. The compound for use as a medicament according to any one of claims 1 - 8, wherein R² and R³ are, independently selected from the group consisting of hydrogen, methyl, secondary butyl, benzyl, or, together with the C atom to which they are attached, provide a C₅-C₆ ring.

10. The compound for use as a medicament according to claim 9, wherein R² and R³ are independently selected from hydrogen or methyl.

11. The compound according to any one of claims 1 - 10, wherein R⁴ is selected from hydrogen, hydroxyl, amino, azido or halo

12. A pharmaceutical composition comprising the compound according to any one of claims 1 - 11 and a pharmaceutically acceptable carrier, diluent or salt.

13. The use of a compound according to any one of claims 1-11 for the preparation of a medicament for the treatment or prophylaxis of cancer.

14. A compound of formula I : 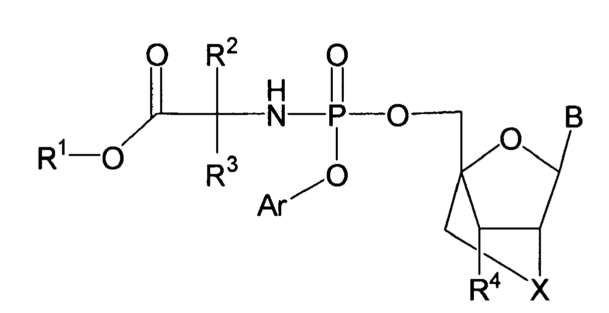 Formula I
wherein:
B, Ar, X, R², R³, R⁴ are as defined in any one of the preceding claims;
wherein R¹ is benzyl.

15. A method for the preparation of the compound according to claim 14, said method comprising reacting of a compound of formula II : 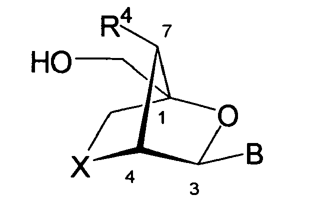 Formula II, wherein B, X and R⁴ are as according to any one of the preceding claims, and wherein the compound of formula B is in the beta-D configuration;
with a compound of formula (III): 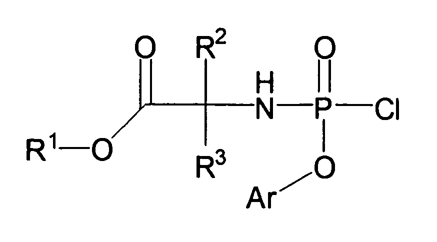 Formula III, wherein Ar, R², and R³ are as defined in any one of the preceding claims and R¹ is benzyl.
to produce the compound according to claim 14.

## Patentansprüche

1. Verbindung der Formel I zur Verwendung als Arzneimittel 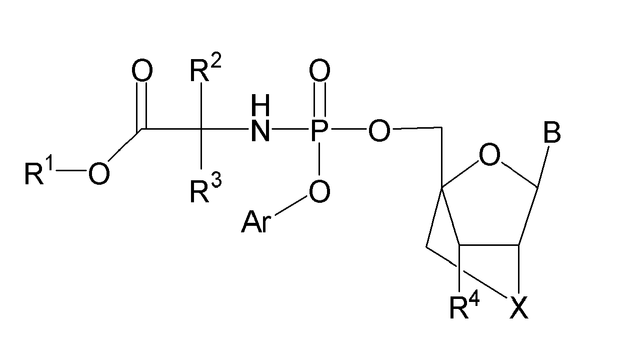 Formel I
worin:
B eine Nukleobase ist;
Ar eine substituierte oder unsubstituierte Arylgruppe ist;
X ausgewählt ist aus der Gruppe, bestehend aus -O-, -S-, >NH und >NR', wobei R' ausgewählt ist aus der Gruppe, bestehend aus Wasserstoff, C₁-C₆-Acyl, C₁-C₆-Alkyl und C₃-C₆-Cycloalkyl;
R¹ ausgewählt ist aus der Gruppe, bestehend aus Alkyl, Cycloalkyl, Aryl und Alkylaryl;
R² und R³ unabhängig voneinander ausgewählt sind aus der Gruppe, umfassend H, primärem, sekundärem, tertiärem C₁₋₆-Alkyl, C₁-C₃-Alkyl C₅-C₇-Aryl, C₃-C₆-Cycloalkyl oder bei gemeinsamer Bildung einer Alkylenkette zusammen mit dem verbundenen C-Atom einen C₃-C₈-carbocyclischen aliphatischen Ring bereitstellen;
R⁴ ausgewählt ist aus der Gruppe, bestehend aus Wasserstoff, Hydroxyl, Amino, Azido, Halogen, fakultativ substituiertem C₁-C₆-Alkyl, fakultativ substituiertem C₃-C₆-Cycloalkyl, fakultativ substituiertem C₁-C₆Alkoxy, Carboxy, Nitril, Nitro, Aryl, Thiol und -Y-CO-Rd, wobei Y ausgewählt ist aus der Gruppe, bestehend aus O, >NH und -S und Rd ausgewählt ist aus der Gruppe, bestehend aus NH₂, OH, C₁-C₆-Alkyl und C₃-C₆-Cycloalkyl,
und wobei B eine natürliche oder nicht natürliche Nukleobase ist, ausgewählt aus der Gruppe, bestehend aus Adenin, Cytosin, 5-Methylcytosin, Isocytosin, Pseudoisocytosin, Guanin, Thymin, Uracil, 5-Bromuracil, 5-Propinyluracil, 5-Propinyl-6-fluoruracil, 5-Methylthiazoluracil, 6-Aminopurin, 2-Aminopurin, Inosin, Diaminopurin, 7-Propinyl-7-deazaadenin, 7-Propinyl-7-deazaguanin und 2-Chlor-6-aminopurin.

2. Verbindung zur Verwendung als Arzneimittel nach Anspruch 1, wobei B Adenin oder ein Adeninanalogon ist.

3. Verbindung zur Verwendung als Arzneimittel nach Anspruch 1 oder 2, wobei Ar eine substituierte oder unsubstituierte monocyclische aromatische Ringgruppierung, wie eine substituierte oder unsubstituierte Phenylgruppe, oder eine kondensierte bicyclische aromatische Ringgruppierung ist.

4. Verbindung zur Verwendung als Arzneimittel nach Anspruch 3, wobei Ar eine substituierte monocyclische aromatische Ringgruppierung oder eine kondensierte bicyclische aromatische Ringgruppierung ist, wobei die Substitution ausgewählt ist aus der Gruppe, bestehend aus Halogen-, Trihalomethyl-, Cyan- und Nitrogruppe.

5. Verbindung zur Verwendung als Arzneimittel nach Anspruch 4, wobei der Substituent ein Halogen, wie Chlor oder Fluor ist.

6. Verbindung zur Verwendung als Arzneimittel nach einem der Ansprüche 1 - 5, wobei X ausgewählt ist aus -O-, -S- und -NH-.

7. Verbindung zur Verwendung als Arzneimittel nach einem der Ansprüche 1 - 6, wobei R¹ ausgewählt ist aus der Gruppe, bestehend aus Methyl, Ethyl, n- oder i-Propyl, n- oder i-Butyl oder Benzyl.

8. Verbindung zur Verwendung als Arzneimittel nach Anspruch 7, wobei R¹ Methyl oder Benzyl ist.

9. Verbindung zur Verwendung als Arzneimittel nach einem der Ansprüche 1 - 8, wobei R² und R³ unabhängig voneinander ausgewählt sind aus der Gruppe, bestehend aus Wasserstoff, Methyl, sekundärem Butyl, Benzyl oder zusammen mit dem daran gebundenen C-Atom einen C₅-C₆-Ring bereitstellen.

10. Verbindung zur Verwendung als Arzneimittel nach Anspruch 9, wobei R² und R³ Unabhängig voneinander ausgewählt sind aus Wasserstoff oder Methyl.

11. Verbindung zur Verwendung als Arzneimittel nach einem der Ansprüche 1 - 10, wobei R⁴ ausgewählt ist aus Wasserstoff, Hydroxyl, Amino, Azido oder Halogen.

12. Pharmazeutische Zusammensetzung, umfassend die Verbindung nach einem der Ansprüche 1 - 11 und einen pharmazeutisch unbedenklichen Träger, ein solches Verdünnungsmittel oder Salz.

13. Verwendung einer Verbindung nach einem der Ansprüche 1-11 zur Herstellung eines Arzneimittels zur Therapie oder Prophylaxe von Krebs.

14. Verbindung der Formel I 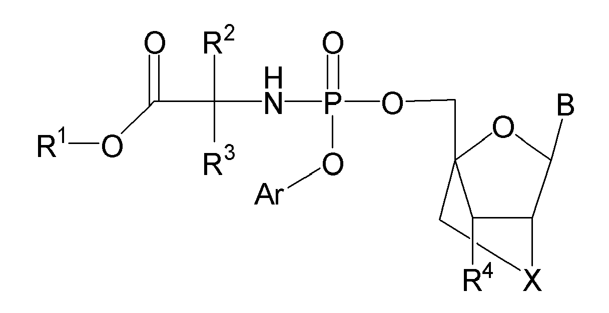 Formel I
worin:
B, Ar, X, R², R³, R⁴ wie in einem der vorstehenden Ansprüche definiert sind;
wobei R¹ Benzyl ist.

15. Verfahren zur Herstellung der Verbindung nach Anspruch 14, wobei das Verfahren die Reaktion einer Verbindung mit der Formel II 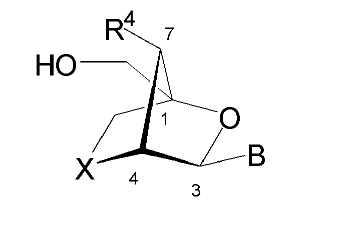 Formel II, wobei B, X und R⁴ gemäß einem der vorhergehenden Ansprüche sind und wobei die Verwendung der Formel B die beta-D-Konfiguration aufweist;
mit einer Verbindung der Formel (III): 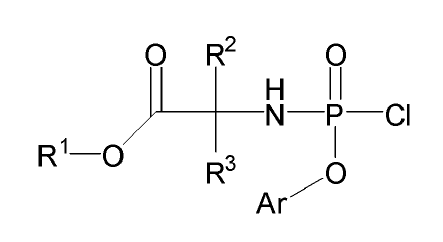 Formel III, wobei Ar, R² und R³ awie in einem der vorstehenden Ansprüche definiert sind und R¹ Benzyl ist;
zur Herstellung der Verbindung nach Anspruch 14 umfasst.

## Revendications

1. Composé de formule I destiné à être utilisé comme médicament 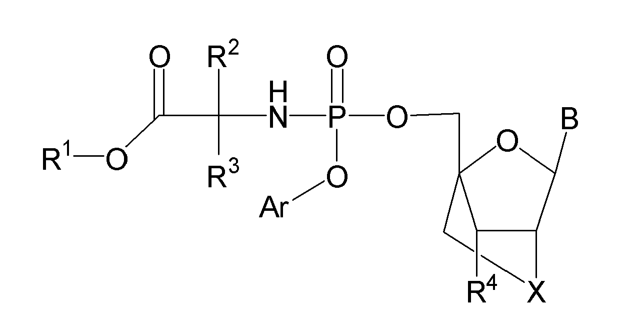 Formule I
où :
B est une nucléobase ;
Ar est un groupement aryle substitué ou non substitué ;
X est choisi dans le groupe consistant en -O-, -S-, >NH et >NR', où R' est choisi dans le groupe consistant en hydrogène, C₁-C₆ acyle, C₁-C₆ alkyle et C₃-C₆ cycloalkyle ;
R¹ est choisi dans le groupe consistant en alkyle, cycloalkyle, aryle et alkylaryle ;
R² et R³ sont choisis indépendamment dans le groupe comprenant H, C₁₋₆ alkyle primaire, secondaire ou tertiaire, C₁-C₃ alkyle, C₅-C₇ aryle, C₃-C₆ cycloalkyle, ou, lorsqu'ils forment ensemble une chaîne alkylène, ils apportent, avec l'atome C auxquels ils sont liés, un cycle aliphatique carbocyclique C₃-C₈ ;
R⁴ est choisi dans le groupe consistant en hydrogène, hydroxyle, amino, azido, halo, C₁-C₆ alkyle éventuellement substitué, C₃-C₆ cycloalkyle éventuellement substitué, C₁-C₆ alkoxy éventuellement substitué, carboxy, nitrilo, nitro, aryle, thiol et -Y-CO-Rd, où Y est choisi dans le groupe consistant en O, >NH et -S, et Rd est choisi dans le groupe consistant en NH₂, OH, C₁-C₆ alkyle et C₃-C₆ cycloalkyle.
et où B est une nucléobase naturelle ou non naturelle choisie dans le groupe consistant en adénine, cytosine, 5-méthylcytosine, isocytosine, pseudoisocytosine, guanine, thymine, uracile, 5-bromouracile, 5-propynyluracile, 5-propyny-6-fluorouracile, 5-méthylthiazoleuracile, 6-aminopurine, 2-aminopurine, inosine, diaminopurine, 7-propyne-7-déazaadénine, 7-propyne-7-déazaguanine et 2-chloro-6-aminopurine.

2. Le composé destiné à être utilisé comme médicament selon la revendication 1, où B est une adénine ou un analogue de l'adénine.

3. Le composé destiné à être utilisé comme médicament selon la revendication 1 ou 2, où Ar est une fraction de cycle aromatique monocyclique substitué ou non substitué, telle qu'un groupement phényle substitué ou non substitué, ou une fraction de cycle aromatique bicyclique fusionné.

4. Le composé destiné à être utilisé comme médicament selon la revendication 3, où Ar est une fraction de cycle aromatique monocyclique substitué ou une fraction de cycle aromatique bicyclique fusionné, où ladite substitution est choisie dans le groupe consistant en groupements halogène, trihalométhyle, cyano et nitro.

5. Le composé destiné à être utilisé comme médicament selon la revendication 4, où le substituant est un halogène, tel que chlore ou fluor.

6. Le composé destiné à être utilisé comme médicament selon l'une quelconque des revendications 1 à 5, où X est choisi parmi -O-, -S- et -NH-.

7. Le composé destiné à être utilisé comme médicament selon l'une quelconque des revendications 1 à 6, où R¹ est choisi dans le groupe consistant en méthyle, éthyle, n- ou i-propyle, n- ou i-butyle, ou benzyle.

8. Le composé destiné à être utilisé comme médicament selon la revendication 7, où R¹ est un méthyle ou un benzyle.

9. Le composé destiné à être utilisé comme médicament selon l'une quelconque des revendications 1 à 8, où R² et R³ sont choisis indépendamment dans le groupe consistant en hydrogène, méthyle, butyle secondaire, benzyle, ou, avec l'atome C auquel ils sont liés, apportent un cycle C₅-C₆.

10. Le composé destiné à être utilisé comme médicament selon la revendication 9, où R² et R³ sont choisis indépendamment parmi hydrogène ou méthyle.

11. Le composé selon l'une quelconque des revendications 1 à 10, où R⁴ est choisi parmi hydrogène, hydroxyle, amino, azido ou halo.

12. Composition pharmaceutique comprenant le composé selon l'une quelconque des revendications 1 à 11 et un véhicule pharmaceutiquement acceptable, un solvant ou un sel.

13. L'utilisation d'un composé selon l'une quelconque des revendications 1 à 11 pour la préparation d'un médicament destiné au traitement ou à la prophylaxie du cancer.

14. Composé de formule I: 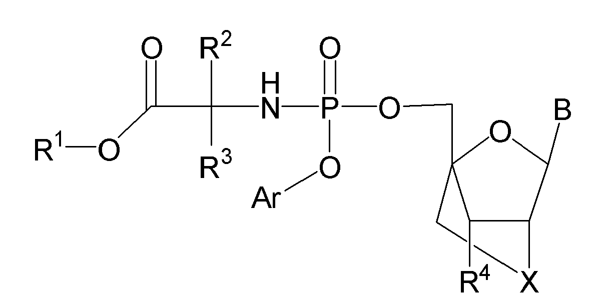 Formule I
où :
B, Ar, X, R², R³, R⁴ sont tels que définis dans l'une quelconque des revendications précédentes ;
où R¹ est un benzyle.

15. Méthode de préparation du composé selon la revendication 14, ladite méthode comprenant la réaction d'un composé de formule II : 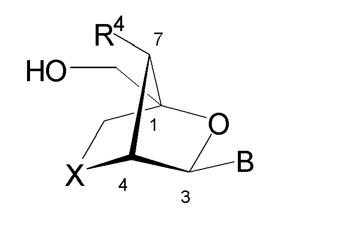 Formule II, où B, X et R⁴ sont tels que définis dans l'une quelconque des revendications précédentes, et où le composé de formule B est en configuration bêta-D ;
avec un composé de formule (III): 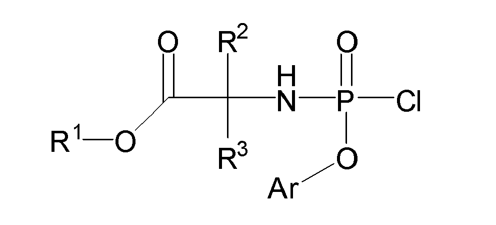 Formule III, où Ar, R² et R³ sont tels que définis dans l'une quelconque des revendications précédentes et R¹ est un benzyle ;
pour produire le composé selon la revendication 14.
